# EUROPÄISCHE PATENTSCHRIFT

(11) **EP 1 060 001 B1**
(45) Veröffentlichungstag und Bekanntmachung des Hinweises auf die Patenterteilung: **28.08.2002**
(21) Anmeldenummer: 98920487.0
(22) Anmeldetag: 25.03.1998
(51) Int. Cl.: B01D 53/72, B01J 20/24, A61L 9/16

(54) **VERFAHREN ZUM SANIEREN VON MIT SCHADSTOFFEN, INSBESONDERE MIT FORMALDEHYD, BELASTETEN INNENRÄUMEN, BZW. GEGENSTÄNDEN**
METHOD FOR CLEANING INTERIOR SPACES OR OBJECTS CONTAMINATED BY HARMFUL SUBSTANCES SUCH AS FORMALDEHYDE IN PARTICULAR
PROCEDE POUR ASSAINIR DES ESPACES INTERIEURS OU DES OBJETS POLLUES AVEC DES SUBSTANCES NOCIVES, NOTAMMENT AVEC DU FORMALDEHYDE

(30) Priorität: 06.03.1998 DE 19809479
(43) Veröffentlichungstag der Anmeldung: 20.12.2000
(73) Patentinhaber: Doppelmayer, Fritz, D-87439 Kempten (DE)
(72) Erfinder: Doppelmayer, Fritz, D-87439 Kempten (DE)
(74) Vertreter: Hutzelmann, Gerhard
(86) Internationale Anmeldenummer: EP9801746
(87) Internationale Veröffentlichungsnummer: WO99044724

(56) Entgegenhaltungen:
- EP-A- 0 204 491
- CA-A- 1 162 904
- US-A- 4 376 807
- US-A- 5 112 652

## Beschreibung

Die Erfindung bezieht sich auf ein Verfahren zum Sanieren von mit Schadstoffen, insbesondere mit Formaldehyd, belasteten Innenräumen bzw. Gegenständen und dauerhaft die Raumluft vor diesen Schadstoffen zu schützen.

Schadstoffe in Innenräumen haben insbesondere durch Verbesserungen im Bereich der Energieeinsparung, die eine erhebliche Reduzierung der Luftwechselrate mit sich bringen, besondere Bedeutung erlangt. Formaldehyd liefert hierzu einen wesentlichen Beitrag. Es ist vor allem in Holzwerkstoffen, insbesondere Spanplatten, Tabakrauch, Parkettversiegelungen, Farben und Lacken, Klebern, Isoliermaterialien und Tapeten enthalten. Das in den Baustoffen enthaltene Formaldehyd entweicht langsam. Bisher sind nur zwei Methoden zur Sanierung von mit Schadstoffen, insbesondere Formaldehyd, belasteten Räumen bekannt. Einerseits werden die belasteten Baustoffe entfernt, was zwar einen dauerhaften Erfolg verspricht, jedoch mit erheblichen Kosten verbunden ist. Eine andere Methode zur Beseitigung der Schadstoffbelastung ist die Beschichtung oder Bekleidung der Schadstoffe emittierenden Materialien mit geeigneten Materialien, so daß die Schadstoffe am Austritt gehindert werden. Die hierbei verwendeten Anstriche unterliegen einem Alterungsprozess, der dazu führt, daß die Wirkungsdauer begrenzt ist. Es besteht zusätzlich die Gefahr, daß durch eine Beschichtung die raumklimatischen Verhältnisse infolge reduzierter bzw. fehlender Feuchtespeicherkapazität ungünstig beeinflusst werden.

In CA-A-1 162 904 und EP-A-0 204 491 wird ein verfahren zur Entfernung von mit Formaldehyd belasteten Innenräumen offenbart, indem ein Gasfilter bestehend aus Woll- bzw. Schafwoll-Fasern ein gesetzt wird.

Ziel der Erfindung ist es, ein Verfahren zur Sanierung schadstoffbelasteter Räume vorzuschlagen, welches die obengenannten Nachteile der hohen Kosten, der zeitlich begrenzten Wirkungsdauer und der negativen Beeinflussung des Raumklimas nicht aufweist.

Dies wird erfindungsgemäß durch die Merkmale der Ansprüche 1 und 2 gelöst.

Die Protein-Fasern (Keratin) nehmen die Schadstoffe auf und bauen diese in einer chemischen Reaktion in ihre Struktur ein, wodurch die Schadstoffe der Raumluft dauerhaft entzogen werden.

Zudem sorgen die Schafwoll-Fasern, die vorzugsweise in einem Verbund als Matte, Platte oder Vließ verwendet werden, für eine ausgezeichnete Schalldämmung. Dadurch wird einerseits eine Schadstoffreinigung der Raumluft, als auch eine hervorragende Geräuschdämmung des Raumes erreicht.

Erfindungsgemäß werden die Schadstoffemittenten mit Schafwoll-Fasern bedeckt.

Dadurch wird wirksam verhindert, daß die Schadstoffe in die Raumluft gelangen können, da diese schon beim Austritt aus dem Emittenten absorbiert werden. Zudem wird dadurch wirkungsvoll eine eventuelle Schwitzwasserbildung auf den Oberflächen der Emittenten verhindert. Der Feuchtigkeitshaushalt der Raumluft wird durch die große Speicherfähigkeit der Keratin-Fasern ausgeglichen.

Erfindungsgemäß wird ein Schadstoffe emittierender Gegenstand, z.B. ein Schrank, an wenigstens einer Seite, vorzugsweise seiner Rückseite mit Schafwoll-Fasern bekleidet.

Hierdurch wird die Schadstoffemission des Gegenstandes langfristig wirksam neutralisiert. Ein Ausmustern des belasteten Gegenstandes ist nicht nötig.

Eine weitere vorteilhafte Ausgestaltung liegt darin, daß zu Matten vernadelte Keratin-Fasern, insbesondere Schafwoll-Fasern verwendet werden.

Dadurch können die Oberflächen der Schadstoffemittenten in einfacher Weise bedeckt werden.

Vorteilhaft ist es auch, wenn durch z.B. walken zu Matten verfilzte Keratin-Fasern, insbesondere Schafwoll-Fasern verwendet werden.

Damit besteht ein weiteres Verfahren zur Herstellung von Matten, mit welchen die Schadstoffemittenten bedeckt werden können.

Es hat sich auch als vorteilhaft erwiesen, wenn zu Platten vernadelte Keratin-Fasern, insbesondere Schafwoll-Fasern verwendet werden.

Hiermit ist es zum Beispiel möglich Stellwände, Trennwände, Decken und/oder Lüftungsanlagen einerseits gegen Schallübertragung zu isolieren und andererseits gleichzeitig eine wirksame Präventivmaßnahme gegen einen zu hohen Schadstoffanteil in der Raumluft z.B. durch Zigarettenrauch oder andere Schadstoffemittenten zu treffen.

Eine weitere vorteilhafte Ausgestaltung liegt auch darin, daß ein verwendeter Verbund aus Schafwoll-Fasern wenigstens mit einer Oberfläche mit einem Trägermaterial verbunden ist.

Dadurch können Verkleidungen, Wände oder dergleichen errichtet werden, die neben der Verkleidungsfunktion zusätzlich eine Schadstoffreinigung der Raumluft bewirken.

Als vorteilhaft hat es sich auch erwiesen, wenn der verwendete Verbund aus aus Schafwoll-Fasern wenigstens einseitig mit Gips kaschiert ist.

Die hierdurch entstehenden Oberflächen können problemlos überstrichen oder tapeziert werden.

Erfindungsgemäß ist es auch vorteilhaft, wenn der verwendete Verbund aus Schafwoll-Fasern wenigstens mit einer Oberfläche mit einer Gipskartonplatte verbunden ist.

Eine aus einer Gipskartonplatte bestehende Oberfläche einer Wand oder dergleichen kann sich bei entsprechender Ausgestaltung auch in einem Feuchtraum befinden und eignet sich besonders zum Bekleben mit Fliesen.

Eine weitere vorteilhafte Ausgestaltungsmöglichkeit liegt darin, daß der verwendete Verbund aus Schafwoll-Fasern wenigstens mit einer Oberfläche mit einer Platte aus Holz, vorzugsweise Weichholz, verbunden ist.

Dadurch werden die Faser-Verbunde mit einem steifen, aber dennoch in gewissem Umfang flexiblen Trägermaterial verbunden und eignen sich deswegen besonders für die Fertigung transportabler Stellwände.

Vorteilhaft ist es auch, wenn mit einem Trägermaterial vermischte Schafwoll-Fasern verwendet werden, wobei wenigstens ein Teil der Fasern bis an die Oberfläche des Verbundes ragen.

Hierdurch wird gewährleistet, daß die Fasern in direkter Verbindung mit der Umgebung steht, aber dennoch ausreichend durch ein Trägermaterial versteift wird.

Es hat sich auch als besonders vorteilhaft erwiesen, wenn zur Schadstoffbeseitigung ein zu Platten geformtes Gemisch aus Schafwoll-Fasern und Gips verwendet wird.

Aus derartige Platten kann eine überstreichbare Wandverkleidung errichtet werden, die trotzdem zu der erfindungsgemäßen Schadstoffsanierung führt.

Weiterhin ist es vorteilhaft, wenn zur Schadstoffbeseitigung ein zu Platten geformtes Gemisch aus Schafwoll-Fasern und Holz, vorzugsweise Weichholz-Platten, verwendet wird.

Hiermit können Stellwände gebildet werden, die eine genügende Steifigkeit aufweisen, die jedoch trotz der Funktion der Schadstoffaufnahme keine direkt sichtbaren Keratin-Fasern enthalten.

## Patentansprüche

1. Verfahren zum Sanieren von mit Schadstoffen, insbesondere mit Formaldehyd, belasteten Innenräumen bzw. Gegenständen und dauerhaft die Raumluft vor diesen Schadstoffen zu schützen, wobei Keratin-Fasern in die belasteten Räume, vorzugsweise in der Nähe der Schadstoffquelle, eingebracht werden, **dadurch gekennzeichnet, daß** die Schadstoffemittenten mit Schafwoll-Fasern bedeckt werden.

2. Verfahren zum Sanieren von mit Schadstoffen, insbesondere mit Formaldehyd, belasteten Innenräumen bzw. Gegenständen und dauerhaft die Raumluft vor diesen Schadstoffen zu schützen, wobei Keratin-Fasern in die belasteten Räume, vorzugsweise in der Nähe der Schadstoffquelle, eingebracht werden, **dadurch gekennzeichnet, daß** ein Schadstoffe emittierender Gegenstand, z.B. ein Schrank, an wenigstens einer Seite, vorzugsweise seiner Rückseite mit Schafwoll-Fasern bekleidet wird.

3. Verfahren nach Anspruch 1 oder 2, **dadurch gekennzeichnet, daß** zu Matten vernadelte Schafwoll-Fasern verwendet werden.

4. Verfahren nach einem der vorangehenden Ansprüche, **dadurch gekennzeichnet, daß** durch z.B. walken zu Matten verfilzte Schafwoll-Fasern verwendet werden.

5. Verfahren nach einem der Ansprüche 1 oder 2, **dadurch gekennzeichnet, daß** zu Platten vernadelte Schafwoll-Fasem verwendet werden.

6. Verfahren nach einem der vorangehenden Ansprüche, **dadurch gekennzeichnet, daß** ein verwendeter Verbund aus Schafwoll-Fasem wenigstens mit einer Oberfläche mit einem Trägermaterial verbunden ist.

7. Verfahren nach Anspruch 6, **dadurch gekennzeichnet, daß** der verwendete Verbund aus Schafwoll-Fasern wenigstens einseitig mit Gips kaschiert ist.

8. Verfahren nach Anspruch 6 oder 7, **dadurch gekennzeichnet, daß** der verwendete Verbund aus Schafwoll-Fasern wenigstens mit einer Oberfläche mit einer Gipskartonplatte verbunden ist.

9. Verfahren nach einem der Ansprüche 6 bis 8, **dadurch gekennzeichnet, daß** der verwendete Verbund aus Schafwoll-Fasern wenigstens mit einer Oberfläche mit einer Platte aus Holz, vorzugsweise Weichholz, verbunden ist.

10. Verfahren nach einem der vorangehenden Ansprüche, **dadurch gekennzeichnet, daß** mit einem Trägermaterial vermischte Schafwoll-Fasern, verwendet werden, wobei wenigstens ein Teil der Fasern bis an die Oberfläche des Verbundes ragen.

11. Verfahren nach Anspruch 10, **dadurch gekennzeichnet, daß** zur Schadstoffbeseitigung ein zu Platten geformtes Gemisch aus Schafwoll-Fasern und Gips verwendet wird.

12. Verfahren nach Anspruch 10, **dadurch gekennzeichnet, daß** zur Schadstoffbeseitigung ein zu Platten geformtes Gemisch aus Schafwoll-Fasern, und Holz, vorzugsweise Weichholz, verwendet wird.

## Claims

1. Process for the restoration of room interiors and/or objects that have been polluted by harmful substances, formaldehyde in particular, which protects the room air against these pollutants on a long-term basis, where keratin fibres are fitted in the polluted rooms, preferably close to the source of the pollutants, **wherein** the emitters of the pollutants are covered with sheep's wool fibres.

2. Process for the restoration of room interiors and/or objects that have been polluted by harmful substances, formaldehyde in particular, which protects the room air against these pollutants on a long-term basis, where keratin fibres are fitted in the polluted rooms, preferably close to the source of the pollutants, **wherein** an object such as a cupboard that emits pollutants is covered with sheep's wool fibres on at least one side, preferably the back.

3. Process according to claim 1 or 2, **wherein** sheep's wool fibres that have been needle-punched into matting are used.

4. Process according to one of the previous claims, **wherein** sheep's wool fibres that are entangled into matting e.g. by fulling are used.

5. Process according to one of claims 1 or 2, **wherein** sheep's wool fibres that have been needle-punched to produce panels are used.

6. Process according to one of the previous claims, **wherein** at least one surface of a composite structure made from sheep's wool fibres that is used is bonded to a substrate material.

7. Process according to claim 6, **wherein** the composite structure made from sheep's wool fibres that is used is laminated with plaster on at least one side.

8. Process according to claim 6 or 7, **wherein** at least one surface of the composite structure made from sheep's wool fibres that is used is bonded to a plasterboard panel.

9. Process according to one of claims 6 to 8, **wherein** at least one surface of the composite structure made from sheep's wool fibres that is used is bonded to a panel made of wood, preferably soft wood.

10. Process according to one of the previous claims, **wherein** sheep's wool fibres combined with a substrate material are used, where at least some of the fibres project to the surface of the composite structure.

11. Process according to claim 10, wherein a combination of sheep's wool fibres and plaster that has been processed into panels is used to eliminate the pollutants.

12. Process according to claim 10, **wherein** a combination of sheep's wool fibres and wood - preferably soft wood - that has been processed into panels is used to eliminate the pollutants.

## Revendications

1. Procédé pour assainir des locaux ou des objets contaminés par des substances nocives, notamment par du formaldéhyde, et protéger durablement l'air ambiant de ces substances nocives, sachant que l'on place des fibres de kératine dans les locaux contaminés, de préférence à proximité du point d'émission de la substance nocive, **caractérisé en ce que** les émetteurs de substances nocives sont recouverts de fibres de laine de mouton.

2. Procédé pour assainir des locaux ou des objets contaminés par des substances nocives, notamment par du formaldéhyde, et pour protéger durablement l'air ambiant de ces substances nocives, sachant que l'on place des fibres de kératine dans les locaux contaminés, de préférence à proximité du point d'émission de la substance nocive, **caractérisé en ce qu'**un objet émettant des substances nocives, par exemple une armoire, est revêtu, au moins d'un côté, de préférence sur l'arrière, de fibres de laine de mouton.

3. Procédé selon la revendication 1 ou 2, **caractérisé en ce que** l'on utilise des fibres de laine de mouton épinglées en nattes.

4. Procédé selon l'une des revendications précédentes, **caractérisé en ce que** l'on utilise des fibres de laine de mouton feutrées en nattes par exemple par foulage.

5. Procédé selon la revendication 1 ou 2, **caractérisé en ce que** l'on utilise des fibres de laine de mouton épinglées en panneaux.

6. Procédé selon l'une des revendications précédentes, **caractérisé en ce qu'**un composé en fibres de laine de mouton utilisé est relié par au moins une surface à un support.

7. Procédé selon la revendication 6, **caractérisé en ce que** le composé en fibres de laine de mouton utilisé est doublé au moins d'un côté avec du plâtre.

8. Procédé selon la revendication 6 ou 7, **caractérisé en ce que** le composé en fibres de laine de mouton utilisé est relié par au moins une surface à un panneau de placoplâtre.

9. Procédé selon l'une des revendications 6 à 8, **caractérisé en ce que** le composé en fibres de laine de mouton utilisé est relié par au moins une surface à un panneau en bois, de préférence en bois tendre.

10. Procédé selon l'une des revendications précédentes, **caractérisé en ce que** l'on utilise des fibres de laine de mouton mélangées à un support, sachant qu'au moins une partie des fibres atteignent la surface du composé.

11. Procédé selon la revendication 10, **caractérisé en ce que**, pour éliminer les substances nocives, on utilise un mélange de fibres de laine de mouton et de plâtre transformé en panneaux.

12. Procédé selon la revendication 10, **caractérisé en ce que**, pour éliminer les substances nocives, on utilise un mélange de fibres de laine de mouton et de bois, de préférence de bois tendre, transformé en panneaux.
